# EUROPEAN PATENT SPECIFICATION

(11) **EP 3 454 926 B1**
(45) Date of publication and mention of the grant of the patent: **19.05.2021**
(21) Application number: 18730958.8
(22) Date of filing: 30.05.2018
(51) Int. Cl.: A61M 15/00, G01P 5/12, A61M 16/00, A61B 5/087

(54) **INHALER DEVICES FOR MONITORING AIRFLOW**
INHALATORVORRICHTUNGEN ZUR ÜBERWACHUNG DES LUFTSTROMS
DISPOSITIFS INHALATEURS POUR SURVEILLER UN FLUX D'AIR

(30) Priority: 30.05.2017 US 201762512631 P
(43) Date of publication of application: 20.03.2019
(60) Divisional application: 21167529.3
(73) Proprietor: Verily Life Sciences LLC, Mountain View, CA 94043 (US)
(72) Inventor: KRASNOW, Benjamin, Mountain View, California 94043 (US)
(74) Representative: Mewburn Ellis LLP
(86) International application number: PCT/US2018/035011
(87) International publication number: WO 2018/222643

(56) References cited:
- EP-A1- 2 638 925
- EP-A1- 2 638 925
- WO-A1-2013/098714
- WO-A2-03/097141
- WO-A2-03/097141
- GB-A- 2 105 046
- US-A- 4 413 514
- US-A- 5 404 871
- US-A- 5 809 997
- US-A- 6 119 684
- US-A1- 2002 000 225
- US-A1- 2002 000 225
- US-A1- 2005 126 562
- US-A1- 2013 172 690
- US-A1- 2013 172 690
- US-A1- 2013 172 690
- US-A1- 2014 261 414
- US-A1- 2014 283 859
- US-A1- 2014 283 859

## Description

### CROSS-REFERENCE TO RELATED APPLICATIONS

This application claims priority to U.S. Provisional Application No. 62/512,631, titled "Inhaler Devices for Monitoring Airflow" and filed on May 30, 2017.

### TECHNICAL FIELD

The present disclosure concerns inhaler devices that are able to monitor airflow before, during, or after administration of a medication.

### BACKGROUND

Respiratory diseases are often treated using medications that must be inhaled by an individual on a regular basis. For example, chronic obstructive pulmonary disease (COPD) can be treated with a dry powder, while asthma can be treated with an aerosol mist of liquid particles. Accordingly, inhaler devices have been created that make it convenient for individuals to inhale their doses of medication. An inhaler device may include, for example, an internal reel of thirty packets of medication or a canister that contains an aerosol mist under pressure.

However, when individuals misuse their inhaler devices, they typically do not receive the proper dose of medication. For instance, if an individual does not inhale forcefully enough, then the medication may become stuck in the throat where it is less effective. Because conventional inhaler devices do not monitor airflow, the individuals who use these conventional inhaler devices (as well as researchers and medical professionals, such as physicians and nurses) do not know how well the medication is being administered. This makes respiratory diseases difficult to treat.

US2014/261414 A1 discloses an inhalation device, control method and computer program The inhalation device comprises a first sensor arranged to generate an inhalation flow signal and a second sensor arranged to generate an exhalation flow signal.

WO2013/098714 A1 discloses an inhalation device configured such that inhaled and exhaled gas are communicated via different paths, the device comprising a flow sensor arranged in an exhaled gas flow path for providing signals relating to the characteristics of the exhaled gas.

US5809997 A discloses an electronic medication chronology device.

### BRIEF DESCRIPTION OF THE DRAWINGS

Various features and characteristics of the technology will become more apparent to those skilled in the art from a study of the Detailed Description in conjunction with the drawings. Embodiments of the technology are illustrated by way of example and not limitation in the drawings, in which like references may indicate similar elements.
Figure 1 depicts an inhaler device that includes a flow sensor disposed within a flow channel.
Figure 2 illustrates how the flow sensor can be attached to thin axial wires using standard soldering techniques, and then securably affixed within the flow channel 104 of the inhaler device.
Figure 3 depicts a flow sensor that is disposed within the mouthpiece of an inhaler device.
Figure 4 is a diagrammatic illustration of an electrical circuit that can be used to pass current through a thermistor, thereby causing its temperature to increase due to resistive heating.
Figure 5 depicts an inhaler device that includes one or more electrodes disposed along the mouthpiece.
Figure 6A depicts an inhaler device that includes an electronics compartment.
Figure 6B is a block diagram of the electronics compartment, which can be configured to acquire, parse, and/or analyze data generated by the inhaler device.
Figure 7 depicts an example of a network environment that includes an inhaler device, a mobile phone, and a network-accessible server system.
Figure 8 depicts a flow diagram of a process for monitoring the airflow through a flow channel of an inhaler device.
Figure 9 depicts a flow diagram of a process for measuring the total volume of air inspired or expired by the lungs.
Figure 10 is a block diagram illustrating an example of a processing system in which at least some operations described herein can be implemented.

The drawings depict various embodiments for the purpose of illustration only. Those skilled in the art will recognize that alternative embodiments may be employed without departing from the principles of the technology. Accordingly, while specific embodiments are shown in the drawings, the technology is amenable to various modifications.

### DETAILED DESCRIPTION

The present invention is defined in the appended independent claim 1. Preferred embodiments of the invention are matter of the dependent claims.

Metered dose inhalers are handheld inhaler devices that deliver a specific amount of medication in aerosol form, rather than as a pill or capsule. Dry powder inhalers, meanwhile, are handheld inhaler devices that deliver a specific amount of medication in dry powder form. Although embodiments may be described in the context of either metered dose inhalers or dry powder inhalers, the features described herein can be used with either type of inhaler device.

Many respiratory diseases require the use of metered dose inhalers and/or dry powder inhalers. Metered dose inhalers are typically intended to provide immediate relief while dry powder inhalers are typically intended to improve long-term outcomes, so compliance with a medication regimen is especially important. However, monitoring compliance is often difficult for a variety of reasons.

For example, if a medical professional wishes to check the status of a respiratory disease, the individual affected by the respiratory disease must travel to a medical facility for a lung capacity check. To better understand disease progression, the medical professional may ask the individual to visit the medical facility on a periodic basis (e.g., daily, weekly, or monthly). But making frequent trips can be inconvenient, in particular because a lung capacity check is a simple test that does not take much time to complete. Frequent trips can also be particularly burdensome if the individual is suffering from an advanced form of a serious respiratory disease.

Introduced here, therefore, are low-cost, disposable inhaler devices configured to perform airflow monitoring. More specifically, an inhaler device can include a robust airflow sensor and a flow control valve that allows the inhaler device to provide feedback regarding inhalation effort before, during, or after the administration of medication. In some embodiments the feedback is transmitted to a computing device in real time, while in other embodiments the feedback is recorded to a local memory and then subsequently transmitted to the computing device. Examples of computing devices include wearable electronic devices (e.g., fitness trackers and watches), mobile phones, laptop computers, computer servers, etc. Some embodiments of the inhaler device also support a lung capacity check functionality (also referred to as a "spirometry functionality"). Thus, the inhaler device may also be configured to provide feedback regarding exhalation effort before, during, or after the administration of medication. These features enable respiratory diseases to be treated more effectively at lower cost.

In the device according to the invention, when an individual places her lips on the mouthpiece of an inhaler device, one or more electrodes can detect that the inhaler device is properly positioned against the lips. The electrode(s) may be arranged in a specified pattern that is known to be indicative of a secure seal by the lips. For example, in some embodiments four electrodes are equally distributed along the exterior perimeter surface of the mouthpiece to ensure that the lips have formed a seal around the mouthpiece, while in other embodiments a single electrode is used to detect the presence of the top lip or the bottom lip.

Embodiments may be described with reference to particular computer programs, system configurations, networks, etc. However, those skilled in the art will recognize that these features are equally applicable to other computer program types, system configurations, network types, etc. For example, while an embodiment may be described in the context of a certain type of inhaler device, those skilled in the art will recognize that the relevant feature is equally applicable when using another type of inhaler device.

Moreover, the technology can be embodied using special-purpose hardware (e.g., circuitry), programmable circuitry appropriately programmed with software and/or firmware, or a combination of special-purpose hardware and programmable circuitry. Accordingly, embodiments may include a machine-readable medium having instructions that may be used to program a computing device to perform a process for parsing airflow data generated by a flow sensor to determine when medication should be administered, whether a medication has been effective in treating a respiratory disease, etc. The instructions may also be used to program a computing device to perform a process for parsing administration data to determine whether an individual has administered a medication in compliance with a medication regimen.

### Terminology

References in this description to "an embodiment" or "one embodiment" means that the particular feature, function, structure, or characteristic being described is included in at least one embodiment. Occurrences of such phrases do not necessarily refer to the same embodiment, nor are they necessarily referring to alternative embodiments that are mutually exclusive of one another.

Unless the context clearly requires otherwise, the words "comprise" and "comprising" are to be construed in an inclusive sense rather than an exclusive or exhaustive sense (i.e., in the sense of "including but not limited to"). The terms "connected," "coupled," or any variant thereof is intended to include any connection or coupling between two or more elements, either direct or indirect. The coupling/connection can be physical, logical, or a combination thereof. For example, devices may be electrically or communicatively coupled to one another despite not sharing a physical connection.

The term "based on" is also to be construed in an inclusive sense rather than an exclusive or exhaustive sense. Thus, unless otherwise noted, the term "based on" is intended to mean "based at least in part on."

The term "module" refers broadly to software components, hardware components, and/or firmware components. Modules are typically functional components that can generate useful data or other output(s) based on specified input(s). A module may be self-contained. A computer program may include one or more modules. Thus, a computer program may include multiple modules responsible for completing different tasks or a single module responsible for completing all tasks.

When used in reference to a list of multiple items, the word "or" is intended to cover all of the following interpretations: any of the items in the list, all of the items in the list, and any combination of items in the list.

The sequences of steps performed in any of the processes described here are exemplary. However, unless contrary to physical possibility, the steps may be performed in various sequences and combinations. For example, steps could be added to, or removed from, the processes described here. Similarly, steps could be replaced or reordered. Thus, descriptions of any processes are intended to be openended.

### Technology Overview

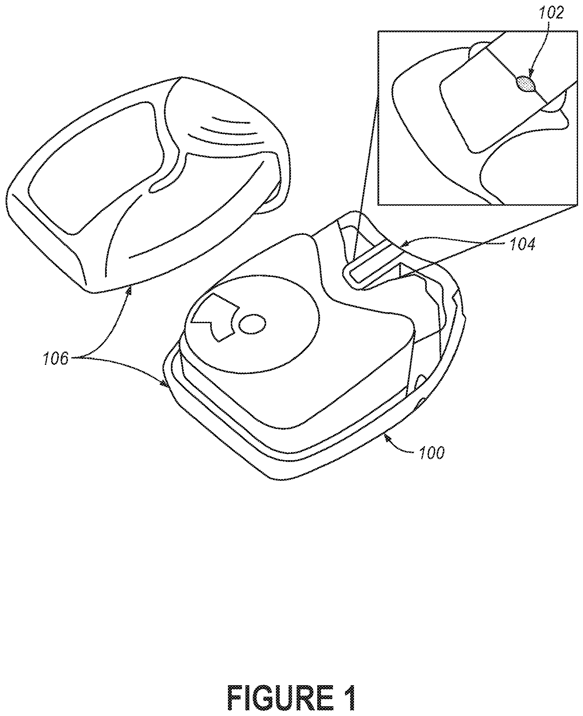

Figure 1 depicts an inhaler device 100 that includes a flow sensor 102 disposed within a flow channel 104. Here, the flow sensor 102 is disposed within the flow channel 104 through which an individual inhales medication. However, in other embodiments the flow sensor 102 is disposed within a separate flow channel through which only air flows. For example, one or more flapper valves may obstruct a main flow channel through which the individual inhales medication until a flow sensor 102 disposed within a secondary flow channel determines that the individual is likely to inhale the medication with sufficient force. In such embodiments, the flow sensor 102 may monitor airflow through the secondary flow channel and then, responsive to a determination that the airflow exceeds a force threshold, generate a signal that causes the flapper valve(s) to be displaced, thereby permitting airflow through the main flow channel. Flapper valve(s) may also obstruct the secondary flow channel. For example, the flapper valve(s) in the main flow channel may work in concert with the flapper valve(s) in the secondary flow channel so that airflow can only travel through one channel at any given point in time.

The flow sensor 102 may be an anemometer that is configured to measure the speed of wind (i.e., breath) traveling through the flow channel 104. For example, the flow sensor 102 could be a hot wire anemometer (also referred to as a "thermistor") that is able to detect airflow through the flow channel 104. Thermistors use a fine wire (generally on the order of several micrometers) that is heated to some temperature above the ambient temperature. In some embodiments, the thermistor includes standard surface-mounted components of the smallest size commonly available (e.g., size 0402 chip thermistors or size 0201 chip thermistors). Such components enable the flow sensor 102 to be embedded within the inhaler device 100 without major changes in device design.

The inhaler device 100 also includes a structural body 106 that includes a recess for holding a container of medication (also referred to as a "medicament") to be inhaled by an individual (also referred to as the "subject" of a medication regimen). For example, in some embodiments the container is a packet that includes a powdered medicament, while in other embodiments the container is a pressurized canister that includes an aerosol medicament. Thus, the recess may take the form of an internal cavity within the structural body 106 or a receptacle interface adapted to receive pressurized canisters.

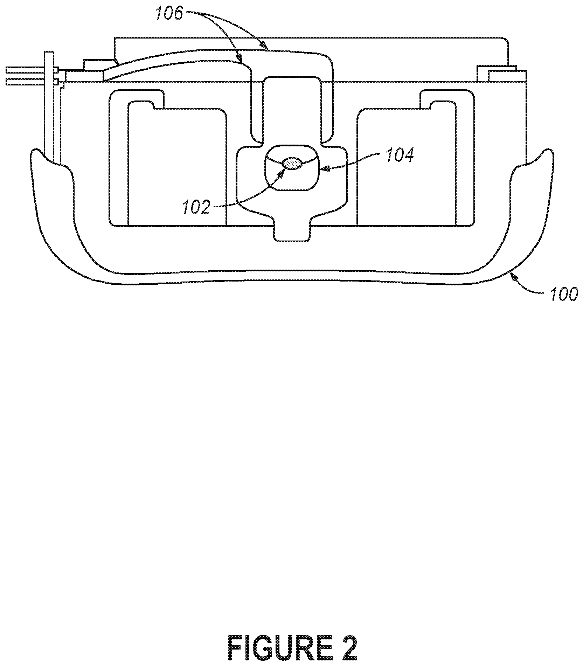

Figure 2 illustrates how the flow sensor 102 can be attached to thin axial wires 106 using standard soldering techniques, and then securably affixed within the flow channel 104 of the inhaler device 100. When an individual draws air (and, in some instances, medication) through the flow channel 104 by inhaling, the air flowing through the flow channel 104 passes over and around the flow sensor 102. Because the axial wires 106 are thin, most thermal leakage out of the flow sensor 102 is into the air rather than through the axial wires 106.

Air flowing through the flow channel 104 will typcally cool the flow sensor 102. In some embodiments, the flow sensor 102 is a thermistor. A thermistor is a type of negative coefficient resistor whose resistance is dependent on temperature. Accordingly, the resistance of a thermistor will go down as the temperature of the thermistor rises. Because the electrical resistance of most metals is dependent on temperature, the relationship between airflow speed and thermistor resistance can be readily established by the inhaler device 100 or some other computing device based on data generated by the flow sensor 102.

The configuration shown here also enables the inhaler device 100 to support airflow sensing functionality without requiring the addition of any moving parts (e.g., flapper valves) that would complicate the design of the inhaler device 100. Moreover, the addition of one or more anemometers to an inhaler device is often very inexpensive.

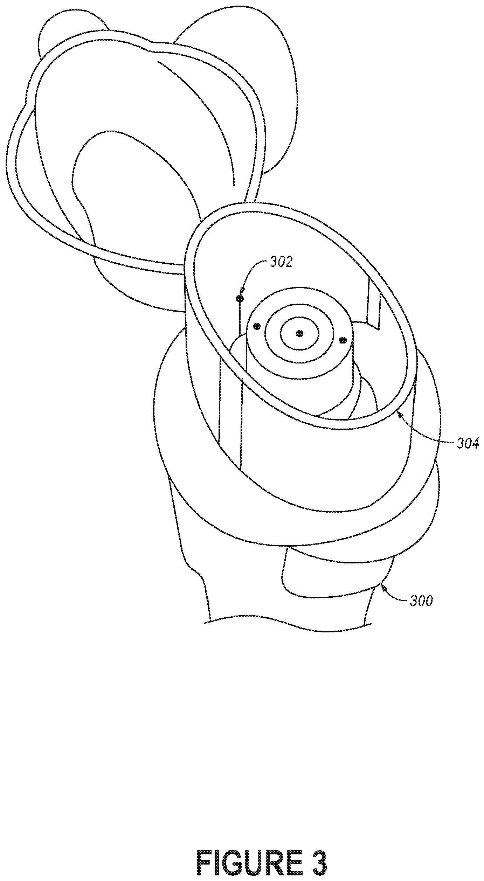

Figure 3 depicts a flow sensor 302 that is disposed within the mouthpiece 304 of an inhaler device 300. While the flow sensor 302 shown here will be positioned proximate to an individual's mouth during use, the flow sensor 302 could be placed anywhere in or near the flow channel through which medication is delivered. Because the inhaler device 300 could be a metered dose inhaler or a dry power inhaler, the medication could be in the form of a powder, an aerosol, etc.

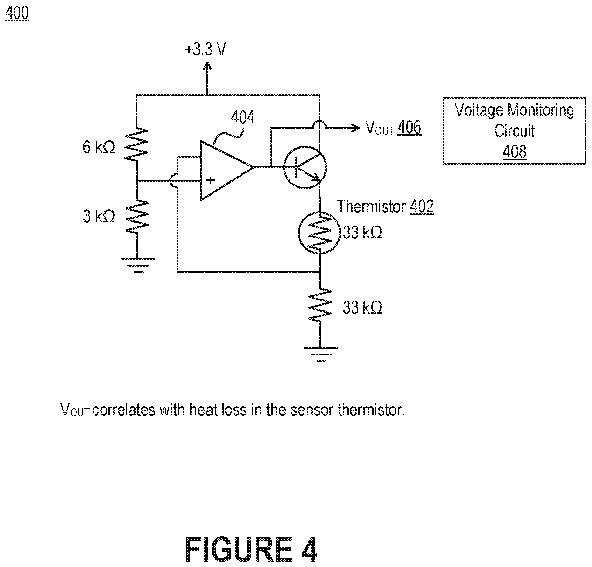

Figure 4 is a diagrammatic illustration of an electrical circuit 400 that can be used to pass current through a thermistor 402, thereby causing its temperature to increase due to resistive heating. The thermistor 402 may be, for example, the flow sensor 102 of Figures 1-2 or the flow sensor 302 of Figure 3. The electrical circuit 400 can estimate the temperature of the thermistor 402 by monitoring the resistance of the thermistor 402. In fact, certain characteristics of the airflow (e.g., speed and volume) can be determined based on the change in resistance of the thermistor 402. For example, at higher airspeeds, the temperature of the thermistor 402 will generally be lower for a fixed current.

According to the invention, the device implements the electrical circuit 400 by using an operational amplifier (also referred to as an "op-amp" or "opamp") 404 to pass a fixed current through the thermistor 402. Because the op-amp 404 controllably varies its output voltage to maintain a constant current, the output voltage (Vₒᵤₜ) 406 of the op-amp 404 will indicate changes in the resistance of the thermistor 402. Said another way, Vₒᵤₜ 406 correlates with heat loss in the thermistor 402. Changes in the resistance of the thermistor 402 (and thus changes in Vₒᵤₜ 406) can be used to estimate changes in air temperature and airflow speed. A voltage monitoring circuit 408 can be configured to monitor Vₒᵤₜ 406, identify a variation in Vₒᵤₜ 406 that exceeds a certain threshold, and cause an airflow property to be estimated based on the variation. The variation may be caused by an inhalation or an exhalation.

Note, however, that accurately determining the airflow based on changes in the thermistors resistance also requires that the ambient air temperature be known. Because the use cases of an inhaler device are inherently discrete (i.e., an individual opens the inhaler device, draws air through a flow channel, and then closes the inhaler device), the thermistor 402 itself can be used to determine the ambient air temperature before the fixed current is applied by the op-amp 404. For example, the electrical circuit 400 may measure the ambient temperature immediately after an individual detaches a cover from the mouthpiece of an inhaler device.

The first measurement (i.e., measuring for the ambient temperature) can be viewed as a discrete measurement of Vₒᵤₜ 406, while the second measurement (i.e., measuring for airflow speed) can be viewed as a continuous measurement of Vₒᵤₜ 406. Once an individual begins breathing into the inhaler device, Vₒᵤₜ 406 can be continually recorded and plotted as a continuous waveform that can be associated with the temperature and speed of the individual's breath.

This technique enables a single thermistor to measure both the ambient temperature and the temperature of the subsequent airflow. These actions are typically performed by a set of multiple thermistors (e.g., a heated thermistor and an unheated thermistor). Accordingly, the airflow sensing functionality can be enabled without requiring the addition of components that increase the complexity of the inhaler device as a whole. As further described below, the airflow temperature could also be approximated as body temperature (∼98.6°F) when the inhaler device is functioning as a spirometer, which again avoids the addition of component(s) that would conventionally be necessary to measure airflow. The temperature determined during the ambient measurement can also be used throughout an airflow monitoring session as a baseline since any other airflow measurement(s) will occur soon after the ambient temperature is measured.

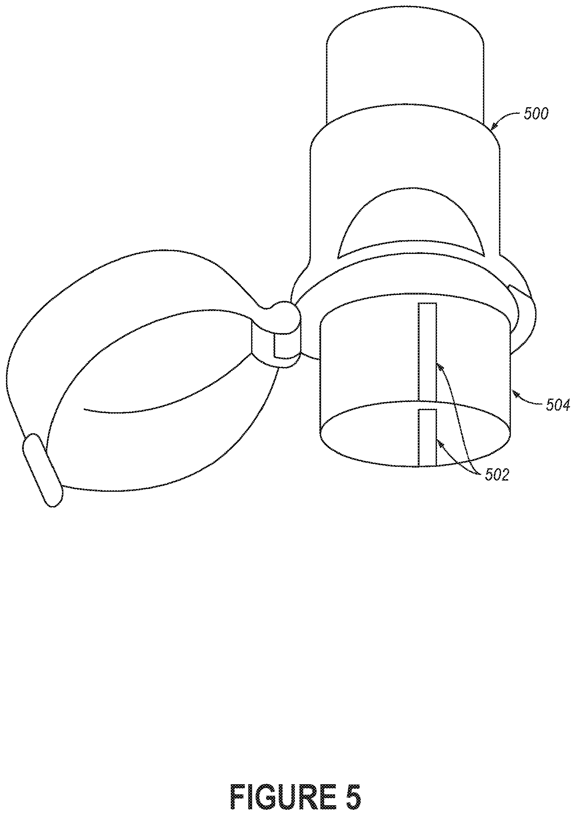

Figure 5 depicts an inhaler device 500 that includes one or more electrodes 502 disposed along the mouthpiece 504. The embodiment shown here includes multiple electrodes arranged along opposite sides of the mouthpiece. However, other embodiments of the inhaler device 500 may include a single electrode or multiple electrodes arranged along a single side of the mouthpiece 504. The electrode(s) 502 are typically embedded within the mouthpiece 504 such that the electrode(s) 502 are exposed along an exterior surface with which an individual's lips make contact. As shown in Figure 5, the electrode(s) 502 could also be exposed along an interior surface of the mouthpiece 504, though the interior surface will generally not come into contact with the individual.

When an individual places her lips on the mouthpiece 504, the electrode(s) 502 can detect that the inhaler device 500 is properly positioned within the mouth. The electrode(s) 502 may be arranged in a specified pattern that is known to be indicative of a secure seal by the individual s lips. For example, in some embodiments four electrodes are equally distributed along the perimeter of the mouthpiece 504 to ensure the lips have formed a seal around the mouthpiece 504, while in other embodiments a single electrode is used to detect the presence of the top lip, the bottom lip, or either oral commissure.

The electrode(s) 502 may also be able to detect and/or record contact events. A contact event indicates that the individual has placed her lips on the corresponding electrode. Contact events may be embodied, for example, as variations in an electrical signal (e.g., a capacitance signal, impedance signal, or resistance signal measured by the electrode(s) 502) that exceed a specified amount.

The inhaler device 500 may only allow medication to be administered upon receiving an indication that a contact event has been recorded by some or all of the electrode(s) 502. For example, in some embodiments the inhaler device 500 includes a locking mechanism that prevents a delivery mechanism (e.g., a trigger button) from causing medication to be dispensed and an electromechanical actuator that, when activated, displaces the locking mechanism. Responsive to receiving a signal from the electrode(s) 502 indicative of a contact event, a processor can transmit an activation signal to the electromechanical actuator. The activation signal causes the electromechanical actuator to displace the locking mechanism, thereby permitting the individual to administer the medication by interacting with the delivery mechanism.

This technique prevents medication from being lost due to accidental triggering of an administration or misunderstanding of how the inhaler device 500 is to be used. Moreover, rather than rely on improvements in subject performance (e.g., due to teaching), the technology instead modifies the design of the inhaler device 500 to improve effectiveness in administering medication.

The electrode(s) 502 are typically intended to be contacted by the upper lip, bottom lip, and/or oral commissure(s). However, an electrode could also be arranged such that the individual can place her tongue against the electrode. For example, the mouthpiece 504 of the inhaler device 500 may include an additional electrode disposed along one of the longitudinal sides (here, the top side or the bottom side) that the individual can place her tongue against before or during administration of the medication. This ensures that the tongue does not occlude airflow (which may include medication in an aerosol form or powder form) through the mouth and into the throat.

Some embodiments of the inhaler device 500 include a mechanical lip sensor rather than, or in addition to, the electrode(s) 502. The mechanical lip sensor can physically prevent the delivery mechanism from being activated unless the individual's lips have applied sufficient pressure to displace the mechanical lip sensor.

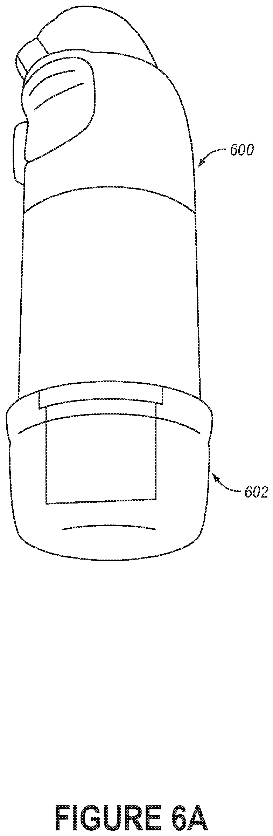

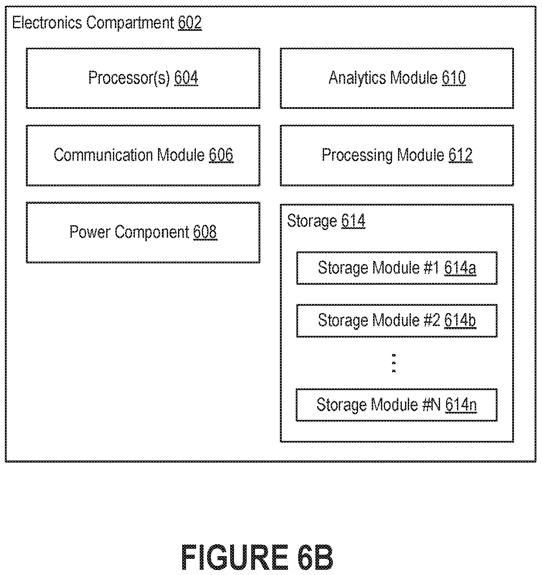

Figure 6A depicts an inhaler device 600 that includes an electronics compartment 602. Figure 6B is a block diagram of the electronics compartment 602, which can be configured to acquire, parse, and/or analyze data generated by the inhaler device 600. The electronics compartment 602 can house a variety of components, including one or more processors 604, a communication modules 606, a memory storage device 614, a power component 608 (e.g., a battery), etc. Embodiments of the electronics compartment 602 can include some or all of these components, as well as other components now described here. For example, the electronics compartment 602 may include an analytics module 610 capable of examining voltage data associated with a flow sensor (e.g., thermistor 402 of Figure 4) to detect variations in airflow speed and/or airflow temperature. As another example, the electronics compartment 602 may include a processing module 612 capable of parsing, formatting, and/or aligning data associated with administrators of medication. For instance, the processing module 612 may filter contact event data prior to storage so that only those contact events associated with administrations of medication are actually recorded.

Additionally or alternatively, the analytics module 610 may be capable of examining contact event data associated with electrode(s) (e.g., electrode(s) 502 of Figure 5) to detect when an individual has placed the inhaler device 600 within her mouth, and thus when medication should be administered. For example, the analytics module 610 could parse the contact event data to discover contact event(s) embodied as variations in an electrical signal (e.g., capacitance variations) exceeding a specified amount, and then associate each contact event with a timestamp generated by a clock module.

Accordingly, the analytics module 610 may be configured to determine when medication should be administered using the voltage data and/or the contact event data. For example, some embodiments of the inhaler device 600 may include one or more valves (e.g., flapper values) that naturally obstruct the flow channel through which an individual inhales medication. By analyzing the voltage data (e.g., Vₒᵤₜ 406 of Figure 4), the analytics module 610 can identify when the individual has begun inhaling and prompt the inhaler device 600 to activate the valves so that medication can flow through the flow channel into the individual's mouth.

As another example, some embodiments of the inhaler device 600 may include an electromechanical actuator that naturally prevents a delivery mechanism (e.g., a trigger button) from being pressed. The electromechanical actuator can include a small solenoid that displaces a locking mechanism for the delivery mechanism. The locking mechanism is typically held in place by a spring. By analyzing the contact event data, the analytics module 610 may activate the electromechanical actuator (thereby allowing the individual to interact with the delivery mechanism) after determining that the individual has placed her lips against the mouthpiece of the inhaler device 600.

The electronics compartment 602 can also include one or more communication modules 606 that are able to send and/or receive data over wireless communication protocols, such as Wi-Fi, Bluetooth®, Near Field Communication (NFC), radio-frequency identification (RFID), wireless Universal Serial Bus (USB), cellular, a proprietary point-to-point protocol, etc. The data may include contact event data, indications of the contact event(s) detected by the analytics module 610, the timestamp(s) identified by the analytics module 610, voltage data, inhaler device information (e.g., connectivity status, battery status, or number of uses remaining), etc.

In some embodiments, the electronics compartment 602 includes a physical connector for communicating with another computing device over a wired channel. Examples of physical connectors include USB Type-A/B/C, Micro-USB, and proprietary ports (e.g., Apple Lightning®). The physical connector may allow transport of data, power, or both. Accordingly, in some embodiments the electronics compartment 602 includes a rechargeable battery that is electrically connected to the wired channel. Additionally or alternatively, the electronics compartment 602 may include a dedicated battery (e.g., a small single cell battery such as a button cell) having a lifespan of weeks, months, or years.

The electronics compartment 602 may be fixedly attached to the inhaler device 600 through the use of permanent/semi-permanent adhesives, fixtures, etc. In such embodiments, the electronics compartment 602 is disposed of along with the inhaler device 600 when all of the medication has been administered or an individual has completed a medication regimen. Alternatively, the electronics compartment 602 may be detachably connected to the inhaler device 600 through the use of a quick release mechanism (e.g., magnets or mechanical features such as clips, tracks, etc.). In such embodiments, the individual could remove the electronics compartment 602 from the inhaler device 600 and place the electronics compartment 602 on another inhaler device. Similarly, the individual could simply remove the electronics compartment 602 before retrieving data (e.g., voltage data, health data, and/or contact event data) stored within its memory. Thus, some embodiments of the electronics compartment 602 may conceal a physical connector when the electronics compartment 602 is affixed to the inhaler device 600.

Some embodiments of the electronics compartment 602 are able to provide visual, audible, and/or tactile notifications. For example, the electronics compartment 602 may include one or more light sources (e.g., light-emitting diodes) that are able to provide visual notifications. The light source(s) may pulse red when an individual is due to administer medication, blue when the electronics compartment 602 is communicatively connected to another computing device (e.g., a mobile phone), and green when the electronics compartment 602 has successfully uploaded data to the other computing device. As another example, the electronics compartment 602 could include one or more haptic actuators able to generate forces, vibrations, or motions. As yet another example, the electronics compartment 602 could include one or more speakers for audibly delivering instructions for administering medication, feedback regarding past administrators or respiratory disease progression, etc. The electronics compartment 602 may also include one or more microphones that, together with the speaker(s), enables the individual to converse with another person (e.g., a pharmacist able to provide instructions for administering medication, a nurse able to monitor adherence to a medication regimen, etc.).

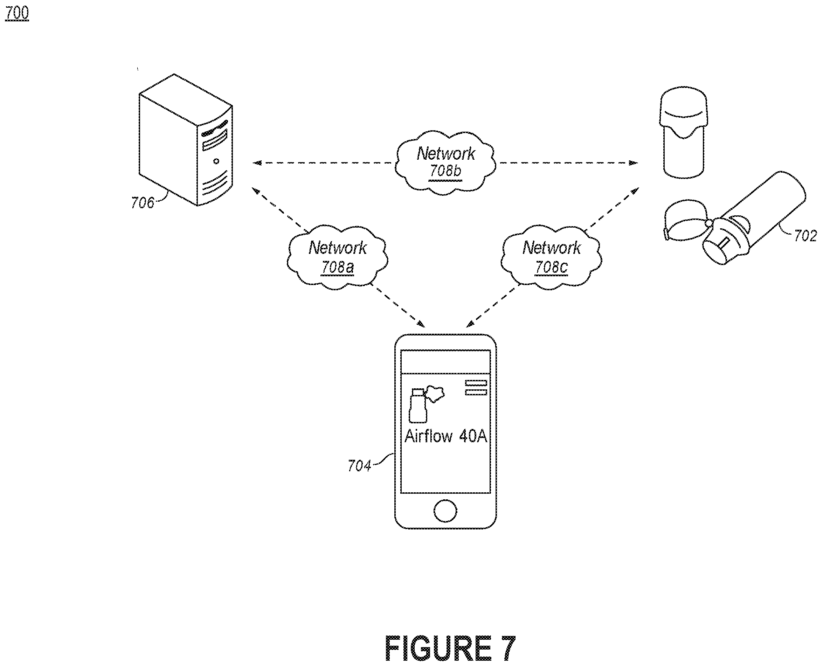

Figure 7 depicts an example of a network environment 700 that includes an inhaler device 702, a mobile phone 704, and a network-accessible server system 706. The inhaler device 702, mobile phone 704, and/or server system 706 can be connected via one or more computer networks 708a-c, which may include the Internet, personal area networks (PANs), local area networks (LANs), wide area networks (WANs), metropolitan area networks (MANs), cellular networks, etc. Additionally or alternatively, the inhaler device 702, mobile phone 704, and/or server system 706 may communicate with one another over a short-range communication protocol, such as Bluetooth® or NFC.

The inhaler device 702 can continually or periodically transmit data associated with administrations of medication to a computer program executing on the mobile phone 704. In some embodiments, the inhaler device 702 automatically syncs with the computer program so long as the inhaler device 702 and the mobile phone 704 are communicatively coupled via a Bluetooth® communication channel. Additionally or alternatively, the inhaler device 702 may periodically sync with the computer program at a specified time (e.g., hourly, daily, or weekly), when available memory on the inhaler device 702 falls below a specified threshold, when the inhaler device 702 and the mobile phone 704 are communicatively coupled to the same network, etc.

Those skilled in the art will recognize that a mobile phone has been used for the purpose of illustration only. Other examples of computing devices that can be communicatively connected to the inhaler device 702 include tablet computers, laptop computers, desktop computers, computer servers, network-connected ("smart") home appliances (e.g., televisions, speakers, and assistant devices), game consoles, mobile gaming devices, television-connected devices (e.g., streaming players and Blu-ray Disc™ players), wearable devices (e.g., watches and fitness trackers), virtual/augmented reality systems (e.g., head-mounted displays), etc.

Generally, the inhaler device 702 is responsible for generating data associated with administrations of medication by an individual. The administration data may include contact events detected by electrode(s) disposed along a mouthpiece of the inhaler device 702, timestamps associated with the contact events, capacitance measurements indicating duration of usage, voltage measurements corresponding to a thermistor disposed within a flow channel of the inhaler device 702, flow profile(s) indicating breath temperature/speed/duration, dosing measurements indicative the date and/or time of administration, lung capacity measurements, etc. The inhaler device 702 may also be configured to track the number of doses remaining, determine the appropriate dosing amounts, detect environmental characteristics (e.g., smog level, pollution level, allergen levels), etc.

As noted above, the inhaler device 702 can be configured to transmit the administration data to the mobile phone 704 for further analysis. For example, upon receiving the health data, the mobile phone 704 may compare timestamp(s) corresponding to contact event(s) to ad administration schedule associated with a medication regimen that requires administration of the medication, and then determine a compliance status based on the comparison. The mobile phone 704 may also generate a notification that specifies the compliance status and, in some instances, transmit the notification to another computing device associated with another individual. The other individual could be, for example, a medical professional, researcher, or family member of the individual responsible for administering the medication.

Secure communication between the inhaler device 702 and the mobile phone 704 can be ensured by having these computing devices complete a key exchange. Thus, the inhaler device 702 can encrypt data and transmit the encrypted data to the mobile phone 704 (e.g., over a Bluetooth® communication channel). The mobile phone 704 can then decrypt the encrypted data using a corresponding key. In some embodiments a similar technique is used to encrypt and decrypt data transmitted by the mobile phone 704 to the inhaler device 702, while in other embodiments no communications are delivered back to the inhaler device 702 (i.e., the inhaler device 702 operates in transmit-only mode). Keys can be exchanged between the inhaler device 702 and the mobile phone 704 in several different ways. For example, an individual may use the mobile phone 704 to scan a machine-readable code (e.g., a Quick Response (QR) code or a bar code) located on the packaging of the inhaler device 702 or on the inhaler device 702 itself.

An individual can then review the data on the mobile phone 704. For example, an individual may view an interface that indicates whether the individual's lips have formed a secure seal around the mouthpiece. As another example, the individual may review certain metrics that indicate whether administration of a medication in compliance with a medication regimen has slowed or halted progression of a respiratory disease. The network-accessible server system 706 could also transmit some or all of the data to another computing device for review by another individual. For example, a medical professional (e.g., a physician or a nurse) may wish to periodically analyze airflow speed or total lung capacity in order to determine whether a current medication regimen is effective. As another example, a physician, insurance provider, or inhaler manufacturer may monitor the effectiveness of a given medication across a group of patients to ascertain effectiveness in treating certain respiratory conditions.

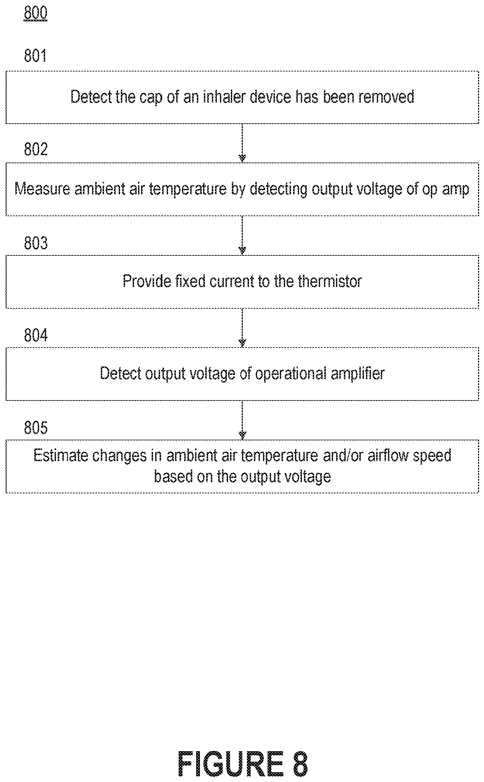

Figure 8 depicts a flow diagram of a process 800 for monitoring the airflow through a flow channel of an inhaler device. An inhaler device can initially detect that a cap has been removed from a mouthpiece (step 801). For example, some embodiments of the inhaler device include optical sensor(s) that detect whether the cap is disposed between an emitter and a detector, while other embodiments include mechanical sensor(s) (e.g., latches and other pressure mechanisms) that detect pressure caused by the cap when it is affixed to the mouthpiece.

The inhaler device can measure the ambient air temperature by detecting the output voltage of an op-amp connected to a thermistor disposed within the flow channel of the inhaler device (step 802). Generally, this is a single (i.e., discrete) measurement taken during or shortly after removal of the cap from the mouthpiece. However, a series of measurements could also be taken during or shortly after removal of the cap from the mouthpiece. For example, the output voltage of the op-amp may be measured multiple times in quick succession and these measurements may be averaged to establish a single average output voltage for determining the ambient air temperature.

The inhaler device can then cause the op-amp to provide a fixed current to the thermistor (step 803). Because the op-amp will alter its output voltage in an attempt to maintain the fixed current, changes in the output voltage of the op-amp (e.g., Vₒᵤₜ 406 of Figure 4) will correspond to changes in the resistance of the thermistor. Said another way, the output voltage of the op-amp correlates with heat loss in the thermistor. Accordingly, the inhaler device can detect the output voltage of the op-amp (step 804), and then estimate the corresponding changes in ambient air temperature and/or airflow speed based on the output voltage (step 805). [PAT(2]

As noted above, the second measurement of the output voltage can be viewed as a continuous measurement of the output voltage of the op-amp. When an individual begins breathing into the inhaler device, the output voltage will begin to vary as the temperature of the air passing over the thermistor varies. Thus, the output voltage could be continually recorded and plotted as a continuous waveform. This waveform can be used to estimate changes in the ambient air temperature and airflow speed (i.e., the speed of the individual's breath during an inhalation or exhalation).

Some embodiments of inhaler devices also include the ability to measure, record, and transmit respiratory data (e.g., respiratory rate and total lung capacity). Said another way, some embodiments of inhaler devices include a spirometer functionality.

To function as a spirometer, an inhaler device may include a one-way valve (also referred to as a "flapper valve") that is activated by the air pressure produced by an individual's lungs. When the individual inhales (thereby producing a pressure lower than the ambient pressure), the one-way valve moves such that the mouthpiece of the inhaler device is connected to the medication chamber and medication can be introduced into the airstream that travels through a primary flow channel. However, when the patient exhales (and produces a pressure higher than the ambient pressure), the one-way valve can swing shut and direct the airflow through a secondary flow channel that bypasses the medication chamber and directs the airflow out of the inhaler device.

According to the invention, the secondary flow channel includes a thermistor that detects variations in airflow. Total lung capacity can be estimated based on these variatins in airflow. The position of the one-way valve could be monitored by capacitive, inductive, mechanical, optical, or magnetic sensing. In some embodiments, a processor uses the position of the one-way valve to control the heating and/or sensing of the thermistor. For example, the thermistor may be configured to only output voltage data in response to a determination that the one-way valve is presently allowing air to travel through the secondary flow channel.

Alternatively, multiple one-way valves could be used to ensure that air is drawn out of the medication chamber of the inhaler device and into the secondary flow channel of the inhaler device. These one-way valves, which may be comprised of thin rubber, may open only when there is a pressure differential in the correct direction.

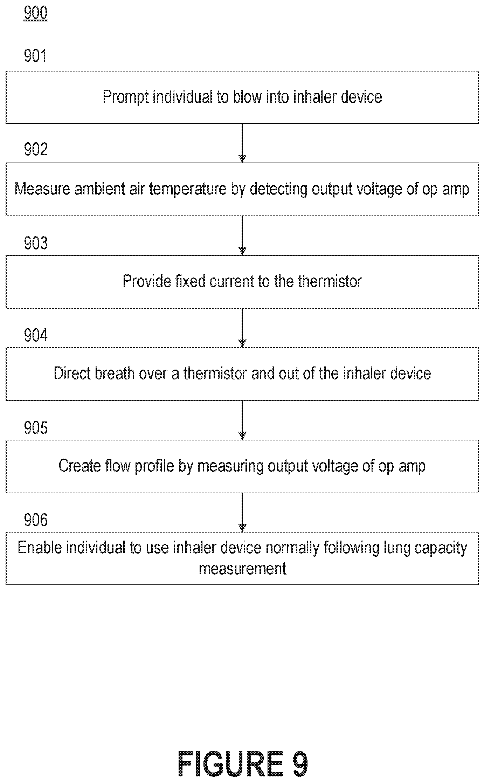

Figure 9 depicts a flow diagram of a process 900 for measuring the total volume of air inspired or expired by the lungs. The inhaler device can initially prompt an individual to blow into the inhaler device (step 901). For example, the inhaler device may be able to visually notify the individual via a display or light-emitting diode(s), audibly notify the individual via a speaker, or tactilely notify the individual via haptic actuator(s). Note that another computing device (e.g., a mobile phone) could also or instead be responsible for prompting the individual to blow into the inhaler device. Steps 902-903 of Figure 9 are largely identical to steps 802-803 of Figure 8.

As noted above, some embodiments of the inhaler device include a one-way valve that directs the individual's breath over a thermistor and out of the inhaler device (step 904). According to the invention, the inhaler device includes a main flow channel through which medication is inhaled and a secondary flow channel through which air is exhaled. The secondary flow channel may extend through the inhaler device such that the individual's breath exits the secondary flow channel an enters the ambient environment.

Because the op-amp controllably varies its output voltage in an attempt to maintain the fixed current, variations in the output voltage can be used to identify instances of heat loss in the thermistor. The inhaler device can create a flow profile by measuring the output voltage of the op-amp responsible for supplying the fixed current to the thermistor (step 905). The flow profile can also be used to estimate the total lung capacity of the individual. For example, the flow profile can be charted as a pneumotachograph, which plots the volume and flow of air coming in and out of the lungs from one inhalation and one exhalation. From a pneumotachograph, an analytics module can readily discover different features such as the forced vital capacity (FVC), forced expiratory volume in one second (FEV1), forced expiratory flow (FEF), peak expiratory flow (PEF), tidal volume (TV), total lung capacity (TLC), etc.

Moreover, due to the presence of the one-way value in the secondary flow channel, the inhaler device enables the individual to use the inhaler device as would normally occur following the lung capacity measurement (step 906).

Unless contrary to physical possibility, it is envisioned that the steps described above may be performed in various sequences by various computing devices. For example, in some embodiments the analytics module responsible for analyzing voltage data generated by the flow sensor resides entirety on the inhaler device, while in other embodiments the analytics module at least partially resides on another computing device (e.g., a mobile phone that is communicatively coupled to the inhaler device). As another example, process 800 of Figure 8 may be performed several times over an interval of time (e.g., a day, week, or month) to monitor compliance with a medication regimen.

Other steps may also be included in some embodiments. For example, if the analytics module determines that the compliance level has fallen below a certain threshold (e.g., due to the individual missing consecutive doses or multiple doses within a certain timespan), the analytics module may cause a notification to be generated. The notification could be delivered to the individual, a medical professional, an insurance provider, a researcher, etc. Moreover, the notification may be in the form of an email message, a text message, a push notification, an automated voice message, etc.

As another example, the inhaler device could be communicatively coupled to another computing device (e.g., a mobile phone) that allows data (e.g., voltage data, health data, or contact event data) to be associated with geographical coordinates that specify a location. This process, which is known as "geotagging," permits information relevant to the administration of the medication to be retrieved and associated with individual administrations of medication. Examples of such information include the smog level, pollution level, allergen level, etc.

### Processing Systems

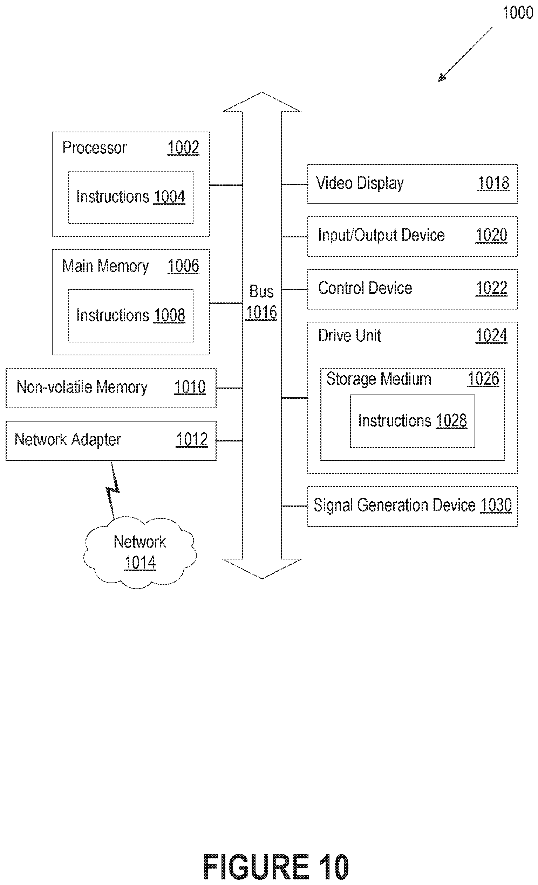

Figure 10 is a block diagram illustrating an example of a processing system 1000 in which at least some operations described herein can be implemented. For example, at least some components of the processing system 1000 may be hosted on an inhaler device (e.g., inhaler device 600 of Figure 6). As another example, at least some components of the processing system 1000 may be hosted on a computing device that is communicatively coupled to an inhaler device.

The processing system may include one or more central processing units ("processors") 1002, main memory 1006, non-volatile memory 1010, network adapter 1012 (e.g., network interfaces), video display 1018, input/output devices 1020, control device 1022 (e.g., keyboard and pointing devices), drive unit 1024 including a storage medium 1026, and signal generation device 1030 that are communicatively connected to a bus 1016. The bus 1016 is illustrated as an abstraction that represents one or more physical buses and/or point-to-point connections that are connected by appropriate bridges, adapters, or controllers. Therefore, the bus 1016 can include a system bus, Peripheral Component Interconnect (PCI) bus or PCI-Express bus, HyperTransport interface, Industry Standard Architecture (ISA) bus, Small Computer System Interface (SCSI) bus, Universal Serial Bus (USB), Inter-Integrated Circuit (I2C or I2C) bus, or Institute of Electrical and Electronics Engineers (IEEE) standard 1394 bus (also referred to as "Firewire").

The processing system 1000 may share a similar computer processor architecture as that of a desktop computer, tablet computer, personal digital assistant (PDA), mobile phone, game console, music player, wearable electronic device (e.g., a watch or fitness tracker), network-connected ("smart") device (e.g., a television or home assistant device), virtual/augmented reality systems (e.g., a head-mounted display), or another electronic device capable of executing a set of instructions (sequential or otherwise) that specify action(s) to be taken by the processing system 1000.

While the main memory 1006, non-volatile memory 1010, and storage medium 1026 (also called a "machine-readable medium") are shown to be a single medium, the term "machine-readable medium" and "storage medium" should be taken to include a single medium or multiple media (e.g., a centralized or distributed database, and/or associated caches and servers) that store one or more sets of instructions 1028. The term "machine-readable medium" and "storage medium" shall also be taken to include any medium that is capable of storing, encoding, or carrying a set of instructions for execution by the processing system 1000.

In general, the routines executed to implement the embodiments of the disclosure may be implemented as part of an operating system process or a dedicated application, component, program, object, module, or sequence of instructions (which are collectively referred to as "computer programs"). The computer programs typically comprise one or more instructions (e.g., instructions 1004, 1008, 1028) set at various times in various memory and storage devices in a computing device. When read and executed by the one or more processors 1002, the instruction(s) cause the processing system 1000 to perform operations to execute elements involving various aspects of the embodiments.

Moreover, while certain embodiments have been described in the context of fully functioning computing devices, those skilled in the art will appreciate that the various embodiments are capable of being distributed as a software program product in a variety of forms. The disclosure applies regardless of the particular type of machine or computer-readable media used to actually effect the distribution.

Further examples of machine-readable storage media, machine-readable media, or computer-readable media include recordable-type media, such as volatile and non-volatile memory devices 1010, floppy and other removable disks, hard disk drives, optical disks (e.g., Compact Disk Read-Only Memory (CD-ROMS) and Digital Versatile Disks (DVDs)), and transmission-type media, such as digital and analog communication links.

The network adapter 1012 enables the processing system 1000 to mediate data in a network 1014 with an entity that is external to the processing system 1000 through any communication protocol supported by the processing system 1000 and the external entity. The network adapter 1012 can include a network adaptor card, a wireless network interface card, a router, an access point, a wireless router, a switch, a multilayer switch, a protocol converter, a gateway, a bridge, bridge router, a hub, a digital media receiver, a repeater, or any combination thereof.

The network adapter 1012 may include a firewall that governs and/or manages permission to access/proxy data in a computer network, and tracks varying levels of trust between different machines and/or applications. The firewall can be any number of modules having any combination of hardware and/or software components able to enforce a predetermined set of access rights between a particular set of machines and applications, machines and machines, and/or applications and applications (e.g., to regulate the flow of traffic and resource sharing between these entities). The firewall may additionally manage and/or have access to an access control list that details permissions including the access and operation rights of an object by an individual, a machine, and/or an application, and the circumstances under which the permission rights stand.

The technology described herein can be implemented by programmable circuitry (e.g., one or more microprocessors), software and/or firmware, special-purpose hardwired (i.e., non-programmable) circuitry, or a combination of such forms. Special-purpose circuitry can be in the form of one or more application-specific integrated circuits (ASICs), programmable logic devices (PLDs), field-programmable gate arrays (FPGAs), etc.

### Remarks

The foregoing description of various embodiments of the claimed subject matter has been provided for the purposes of illustration and description. It is not intended to be exhaustive or to limit the claimed subject matter to the precise forms disclosed. Many modifications and variations will be apparent to one skilled in the art. Embodiments were chosen and described in order to best describe the principles of the invention and its practical applications, thereby enabling those skilled in the relevant art to understand the claimed subject matter, the various embodiments, and the various modifications that are suited to the particular uses contemplated.

Although the Detailed Description describes certain embodiments and the best mode contemplated, the technology can be practiced in many ways no matter how detailed the Detailed Description appears. Embodiments may vary considerably in their implementation details, while still being encompassed by the specification. Particular terminology used when describing certain features or aspects of various embodiments should not be taken to imply that the terminology is being redefined herein to be restricted to any specific characteristics, features, or aspects of the technology with which that terminology is associated. In general, the terms used in the following claims should not be construed to limit the technology to the specific embodiments disclosed in the specification, unless those terms are explicitly defined herein. Accordingly, the actual scope of the technology encompasses not only the disclosed embodiments, but also all equivalent ways of practicing or implementing the embodiments.

The language used in the specification has been principally selected for readability and instructional purposes. It may not have been selected to delineate or circumscribe the subject matter. It is therefore intended that the scope of the technology be limited not by this Detailed Description, but rather by the claims which follow. Accordingly, the disclosure of various embodiments is intended to be illustrative, but not limiting, of the scpe of the technology as set forth in the following claims.

## Claims

1. An inhaler device (100, 300, 500, 600, 702) for dispensing medication, the inhaler device comprising:
a structural body (106) that includes a recess for holding a container of medication to be inhaled by a subject;
a primary flow channel (104) through which the medication travels when air is drawn through the flow channel by the subject;
a secondary flow channel through which only air travels when air is expelled out during an exhalation by the subject;
a thermistor affixed (102) within the secondary flow channel;
an electrical circuit (400) comprising:
an operational amplifier (404) that passes a fixed current through the thermistor (102);
a voltage monitoring circuit (408) configured to:
monitor an output voltage of the operational amplifier (404),
identify a variation in the output voltage exceeding a certain threshold,
wherein the variation is caused by the operational amplifier (404) attempting to maintain the fixed current, and
estimate an airflow property based on the variation;
a locking mechanism configured to prevent a delivery mechanism from causing the medication to be dispensed from the container;
a mouthpiece (504) that includes an electrode (502) for detecting a presence of the subject's lips on the mouthpiece; and
a controller configured to:
receive a signal from the electrode (502) indicative of a contact event between the electrode (502) and an upper lip, a bottom lip, or an oral commissure,
generate an activation signal that causes the locking mechanism to be displaced, thereby permitting the subject to administer the medication by interacting with the delivery mechanism.

2. The inhaler device of claim 1, wherein the container is a packet that includes a powdered medicament.

3. The inhaler device of claim 1, wherein the container is a pressurized canister that includes an aerosol medicament.

4. The inhaler device of claim 1, wherein the delivery mechanism includes a trigger button that, upon being activated, causes the medication to be dispensed from the container.

5. The inhaler device of claim 1, wherein the electrode (502) is one of multiple electrodes that are exposed through an exterior surface of the mouthpiece (504).

6. The inhaler device of claim 1, further comprising:
an electronics compartment (602) that includes:
a power component (608); and
a communication module (606) configured to wirelessly transmit information to a computing device (704) across a network (708);
wherein the controller is configured to:
acquire voltage data that includes measurements of output voltage of the electrical circuit (400),
parse the voltage data to discover a variation exceeding a certain threshold, and
associate the variation with a timestamp generated by a clock module.

7. The inhaler device of claim 6, wherein the controller is further configured to:
acquire an administration schedule associated with a medication regimen that requires administration of the medication from a memory;
compare the timestamp to the administration schedule; and
determine a compliance status for the subject based on the comparison,
wherein the compliance status is included in the information transmitted to the computing device by the communication module.

8. The inhaler device of claim 1, further comprising:
a flapper valve affixed within the primary flow channel (104);
wherein the controller is further configured to:
acquire voltage data generated by the electrical circuit,
parse the voltage data to discover a variation,
determine that the variation corresponds to a change in airflow speed of at least a certain amount, and
generate a signal to actuate the flapper valve, thereby allowing the subject to inhale the medication through the primary flow channel (104).

## Patentansprüche

1. Inhalatorvorrichtung (100, 300, 500, 600, 702) zur Medikamentenabgabe, wobei die Inhalatorvorrichtung Folgendes umfasst:
einen Strukturkörper (106), der eine Aussparung zum Halten eines Behälters eines Medikaments, das von einem Individuum inhaliert werden soll, umfasst;
einen primären Strömungskanal (104), durch den das Medikament strömt, wenn Luft von dem Individuum durch den Strömungskanal gesaugt wird;
einen sekundären Strömungskanal, durch den nur Luft strömt, wenn Luft während einer Ausatmung durch das Individuum ausgestoßen wird;
einen Thermistor (102), der innerhalb des sekundären Strömungskanals befestigt ist;
eine elektronische Schaltung (400), die Folgendes umfasst:
einen Operationsverstärker (404), der einen festgelegten Strom durch den Thermistor (102) einprägt;
eine Spannungsüberwachungsschaltung (408), die ausgelegt ist, um:
eine Ausgangsspannung des Operationsverstärkers (404) zu überwachen,
eine Schwankung der Ausgangsspannung, die einen bestimmten Schwellwert übersteigt, zu identifizieren, wobei die Schwankung von dem Operationsverstärker (404) verursacht wird, der bestrebt ist, den festgelegten Strom aufrechtzuerhalten, und
eine Luftstromeigenschaft auf Basis der Schwankung zu schätzen;
einen Arretiermechanismus, der ausgelegt ist, um zu verhindern, dass ein Abgabemechanismus die Abgabe des Medikaments von dem Behälter verursacht;
ein Mundstück (504), das eine Elektrode (502) zur Detektion eines Vorhandenseins von Lippen des Individuums auf dem Mundstück umfasst; und
eine Steuerung, die ausgelegt ist, um:
ein Signal von der Elektrode (502) zu empfangen, das ein Kontaktereignis zwischen der Elektrode (502) und einer Oberlippe, einer Unterlippe oder eines Mundwinkels anzeigt,
ein Aktivierungssignal zu erzeugen, das das Verlagern des Arretiermechanismus verursacht, wodurch dem Individuum durch Interaktion mit dem Abgabemechanismus die Verabreichung des Medikaments ermöglicht wird.

2. Inhalatorvorrichtung nach Anspruch 1, wobei der Behälter eine Packung ist, die ein pulverförmiges Medikament umfasst.

3. Inhalatorvorrichtung nach Anspruch 1, wobei der Behälter eine unter Druck stehende Dose ist, die ein Medikament in Aerosolform umfasst.

4. Inhalatorvorrichtung nach Anspruch 1, wobei der Abgabemechanismus einen Auslöseknopf umfasst, der bei Betätigung bewirkt, dass das Medikament aus dem Behälter abgegeben wird.

5. Inhalatorvorrichtung nach Anspruch 1, wobei die Elektrode (502) eine von mehreren Elektroden ist, die durch eine Außenfläche des Mundstücks (504) freigelegt sind.

6. Inhalatorvorrichtung nach Anspruch 1, die außerdem Folgendes umfasst:
ein Elektronikfach (602), das Folgendes umfasst:
eine Leistungskomponente (608); und
ein Kommunikationsmodul (606), das ausgelegt ist, um Informationen drahtlos über ein Netzwerk (708) an eine Computervorrichtung (704) zu übertragen;
wobei die Steuerung ausgelegt ist, um:
Spannungsdaten zu erfassen, die Messungen einer Ausgangsspannung der elektrischen Schaltung (400) umfassen,
die Spannungsdaten zu analysieren, um eine Schwankung zu entdecken, die einen bestimmten Schwellwert übersteigt, und
die Schwankung einem Zeitstempel zuzuordnen, der von einem Taktmodul erzeugt wurde.

7. Inhalatorvorrichtung nach Anspruch 6, wobei die Steuerung außerdem ausgelegt ist, um:
einen Verabreichungszeitplan, der einem Medikationsschema zugeordnet ist, das die Verabreichung des Medikaments erfordert, aus einem Speicher zu erhalten;
den Zeitstempel mit dem Verabreichungszeitplan zu vergleichen; und
einen Einhaltungsstatus für das Individuum auf Basis des Vergleichs zu bestimmen, wobei der Einhaltungsstatus in den Informationen umfasst ist, die von dem Kommunikationsmodul an die Computervorrichtung übertragen werden.

8. Inhalatorvorrichtung nach Anspruch 1, die außerdem Folgendes umfasst:
ein Klappenventil das innerhalb des primären Strömungskanals (104) befestigt ist;
wobei die Steuerung außerdem ausgelegt ist, um:
Spannungsdaten, die von der elektronischen Schaltung erzeugt werden, zu erfassen,
die Spannungsdaten zu analysieren, um eine Schwankung zu entdecken,
zu bestimmen, dass die Schwankung einer Änderung der Luftstromgeschwindigkeit um zumindest ein bestimmtes Ausmaß entspricht, und
ein Signal zu erzeugen, um das Klappenventil zu betätigen, wodurch dem Individuum ermöglicht wird, das Medikament durch den primären Strömungskanal (104) einzuatmen.

## Revendications

1. Dispositif inhalateur (100, 300, 500, 600, 702) pour la distribution de médicament, le dispositif inhalateur comprenant :
un corps structurel (106) qui comprend un évidement pour contenir un récipient de médicament à inhaler par un sujet ;
un canal d'écoulement principal (104) à travers lequel le médicament se déplace lorsque de l'air est aspiré à travers le canal d'écoulement par le sujet ;
un canal d'écoulement secondaire à travers lequel seul de l'air circule lorsque de l'air est expulsé pendant une expiration par le sujet ;
une thermistance fixée (102) dans le canal d'écoulement secondaire ;
un circuit électrique (400) comprenant :
un amplificateur opérationnel (404) qui fait passer un courant fixe à travers la thermistance (102) ;
un circuit de surveillance de tension (408) configuré pour :
surveiller une tension de sortie de l'amplificateur opérationnel (404),
identifier une variation de la tension de sortie dépassant un certain seuil,
dans lequel la variation est provoquée par l'amplificateur opérationnel (404) qui tente de maintenir le courant fixe, et
estimer une propriété d'écoulement d'air sur la base de la variation ;
un mécanisme de verrouillage configuré pour empêcher qu'un mécanisme de délivrance n'amène le médicament à être distribué depuis le récipient ;
un embout buccal (504) qui comprend une électrode (502) pour détecter une présence des lèvres du sujet sur l'embout buccal ; et
un dispositif de commande configuré pour :
recevoir un signal provenant de l'électrode (502) indiquant un événement de contact entre l'électrode (502) et une lèvre supérieure, une lèvre inférieure, ou une commissure orale,
générer un signal d'activation qui amène le mécanisme de verrouillage à être déplacé, permettant ainsi au sujet d'administrer le médicament en interagissant avec le mécanisme de délivrance.

2. Dispositif inhalateur selon la revendication 1, dans lequel le récipient est un sachet qui comprend un médicament en poudre.

3. Dispositif inhalateur selon la revendication 1, dans lequel le récipient est une cartouche pressurisée qui comprend un médicament aérosol.

4. Dispositif inhalateur selon la revendication 1, dans lequel le mécanisme de délivrance comprend un bouton de déclenchement qui, lorsqu'il est activé, amène le médicament à être distribué depuis le récipient.

5. Dispositif inhalateur selon la revendication 1, dans lequel l'électrode (502) est l'une de multiples électrodes qui sont exposées à travers une surface extérieure de l'embout buccal (504).

6. Dispositif inhalateur selon la revendication 1, comprenant en outre :
un compartiment électronique (602) qui comprend :
un composant de puissance (608) ; et
un module de communication (606) configuré pour transmettre sans fil des informations à un dispositif informatique (704) à travers un réseau (708) ;
dans lequel le dispositif de commande est configuré pour :
acquérir des données de tension qui incluent des mesures de tension de sortie du circuit électrique (400),
analyser les données de tension pour découvrir une variation dépassant un certain seuil, et
associer la variation à un horodatage généré par un module d'horloge.

7. Dispositif inhalateur selon la revendication 6, dans lequel le dispositif de commande est en outre configuré pour :
acquérir un programme d'administration associé à un régime médicamenteux qui nécessite une administration du médicament auprès d'une mémoire ;
comparer l'horodatage au programme d'administration ; et
déterminer un état de conformité pour le sujet sur la base de la comparaison,
dans lequel l'état de conformité est inclus dans les informations transmises au dispositif informatique par le module de communication.

8. Dispositif inhalateur selon la revendication 1, comprenant en outre :
une soupape à clapet fixée à l'intérieur du canal d'écoulement primaire (104) ;
dans lequel le dispositif de commande est en outre configuré pour :
acquérir des données de tension générées par le circuit électrique,
analyser les données de tension pour découvrir une variation,
déterminer que la variation correspond à un changement de vitesse d'écoulement d'air d'au moins une certaine quantité, et
générer un signal pour actionner la soupape à clapet, permettant ainsi au sujet d'inhaler le médicament à travers le canal d'écoulement principal (104).
